# EUROPÄISCHE PATENTSCHRIFT

(11) **EP 1 399 114 B1**
(45) Veröffentlichungstag und Bekanntmachung des Hinweises auf die Patenterteilung: **28.03.2007**
(21) Anmeldenummer: 02718160.1
(22) Anmeldetag: 23.02.2002
(51) Int. Cl.: A61K 8/41, A61K 8/46, A61Q 5/10

(54) **MITTEL ZUM FÄRBEN VON KERATINFASERN ENTHALTEND CHINONIMINDERIVATE UND VERFAHREN**
AGENT CONTAINING QUINONIMINE DERIVATIVES AND USED TO COLOUR KERATIN FIBRES, AND ASSOCIATED METHOD
SUBSTANCE CONTENANT DES DERIVES DE QUINONE-IMINE, DESTINEE A COLORER DES FIBRES DE KERATINE ET PROCEDE ASSOCIE

(30) Priorität: 19.06.2001 DE 10129545
(43) Veröffentlichungstag der Anmeldung: 24.03.2004
(73) Patentinhaber: Wella Aktiengesellschaft, 64274 Darmstadt (DE)
(72) Erfinder: PASQUIER, Cécile, CH-1723 Marly (CH); BUCLIN-CHARRIERE, Veronique, CH-1638 Morlon (CH); WYSS, Patrick, CH-1212 Grand-Lancy (CH)
(86) Internationale Anmeldenummer: PCT/EP2002/001925
(87) Internationale Veröffentlichungsnummer: WO 2002/102336

(56) Entgegenhaltungen:
- US-A- 3 041 244
- US-A- 3 963 764
- NAIR V ET AL: "Dipolar cycloaddition of carbonyl ylides to para-quinoneimides: a facile route to bicyclo[3.2.1] and [2.2.1] systems" TETRAHEDRON LETTERS, ELSEVIER SCIENCE PUBLISHERS, AMSTERDAM, NL, Bd. 42, Nr. 10, 4. März 2001 (2001-03-04), Seiten 2045-2046, XP004316786 ISSN: 0040-4039

## Beschreibung

Gegenstand der vorliegenden Erfindung ist ein Mittel zur Färbung von Keratinfasern, wie zum Beispiel Seide, Wolle oder Haaren und insbesondere menschlichen Haaren, welches eine Kombination aus (i) mindestens einem Chinoniminderivat der Formel (I) und (ii) mindestens einem aromatischen Amin oder Phenol enthält, sowie ein Verfahren zum Färben von Keratinfasern unter Verwendung dieses Färbemittels.

Haarfärbemittel werden je nach zu färbender Ausgangshaarfarbe und gewünschtem Endresultat hauptsächlich in den Bereich der Oxidationsfärbemittel oder der Tönungen unterteilt. Oxidationshaarfarben eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe wie z. B. die Nitrofarbstoffe, können aufgrund ihrer geringen Größe in das Haar eindringen und es - zumindestens in den äußeren Bereichen - direkt anfärben. Derartige Tönungen sind sehr haarschonend und überstehen in der Regel 6 bis 8 Haarwäschen. Direktziehende Farbstoffe, insbesondere Nitrofarbstoffe werden ebenfalls oft in oxidativen Färbemitteln zur Erzeugung bestimmter Nuancen beziehungsweise zur Intensivierung der Farbe eingesetzt.

Die Verwendung von Iminen in Haarfärbemittel ist aus der Literatur bekannt. So beschreibt die DE-OS 199 32 565 N-Halogenimine enthaltende Haarfärbemittel. Die dort beschriebenen Verbindungen erfüllen die an Färbemittel für Keratinfasern gestellten Anforderungen jedoch nicht in jeder Hinsicht. Es bestand daher weiterhin ein großer Bedarf für Färbemittel, die unter milden Bedingungen sowohl intensive als auch schonende Färbungen ermöglichen.

Überraschenderweise wurde nunmehr gefunden, dass durch die Verwendung einer Kombination aus (i) mindestens einem Chinonimin der Formel (I) und (ii) mindestens einem Amin und/oder Phenol, auf schonende Weise und unter milden Bedingungen, auch in Abwesenheit von Oxidationsmitteln, intensive Färbungen mit einer großen Nuancenvielfalt ermöglicht werden. Die erfindungsgemäße Kombination ermöglicht zudem reinere Ausfärbungen als mit üblichen Oxidationsfärbemitteln.

Gegenstand der vorliegenden Erfindung ist daher ein Mittel zur Färbung von Fasern (A), wie zum Beispiel Wolle, Seide, Baumwolle oder Haaren und insbesondere menschlichen Haaren, welches durch Vermischen zweier Komponenten (A1) und (A2) hergestellt wird und dadurch gekennzeichnet ist, dass die Komponente (A1) mindestens ein Chinonimin der Formel (1) enthält, und die Komponente (A2) mindestens eine Verbindung aus der Gruppe bestehend aus aromatischen Aminen und Phenolen enthält; 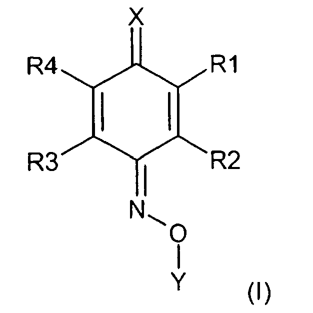 wobei in Formel (I)
**X** gleich Sauerstoff oder einer NR-Gruppe (mit R gleich einer gegebenenfalls substituierten (C₁-C₆)-Alkylgruppe oder einem gegebenenfalls substituierten aromatischen -isozyklischen oder heterozyklischen- Ring) ist;
**Y** gleich einer (C₁-C₆)-Alkylsulfonylgruppe, einer Arylsulfonyl oder einer Acetylgruppe ist;
**R1, R2, R3** und **R4** gleich oder verschieden sein können und unabhängig voneinander Wasserstoff, ein Halogenatom (F, Cl, Br, J), eine (C₁-C₆)-Alkylgruppe, eine Trifluormethylgruppe, eine mit einem Halogenatom (F, Cl, Br, J) substituierte (C₁-C₆)-Alkylgruppe, eine (C₁-C₆)-Hydroxyalkylgruppe, eine (C₂-C₆)-Polyhydroxyalkylgruppe, eine (C₁-C₆)-Aminoalkylgruppe, eine (C₁-C₆)-Alkoxygruppe, eine (C₁-C₆)-Hydroxyalkoxygruppe, eine (C₁-C₆)-Aminoalkoxygruppe, eine (C₁-C₆)-Alkoxy-(C₁-C₆)-alkylgruppe, eine (C₁-C₆)-Alkoxy-(C₁-C₆)-alkoxygruppe, eine (C₁-C₆)-Hydroxyakyl-(C₁-C₆)-aminoalkylgruppe, eine Nitrogruppe, eine Cyanogruppe, eine Carbonsäuregruppe, eine Carbonsäureestergruppe oder ein gegebenenfalls substituiertes aromatisches (-isozyklisches oder heterozyklisches) Ringsystem darstellen, wobei auch zwei benachbarte Reste **R1** bis **R4** gemeinsam einen ankondensiertes aromatisches (isozyklisches oder heterozyklisches) Ringsystem bilden können.

Bevorzugt sind Verbindungen der Formel (I) in denen **X** gleich Sauerstoff ist, **Y** gleich einer Methylsulfonylgruppe, einer Phenylsulfonylgruppe, einer 4-Tolylsulfonylgruppe, einer 4-Chlorphenylsulfonylgruppe, einer Acetylgruppe oder Chloracetylgruppe ist, und **R1, R2, R3** und **R4** gleich oder verschieden sein können und unabhängig voneinander Wasserstoff, ein Halogenatom (F, Cl, Br, J), eine (C₁-C₆)-Alkylgruppe, eine (C₁-C₆)-Hydroxyalkylgruppe, eine (C₁-C₆)-Aminoalkylgruppe, eine (C₁-C₆)-Alkoxygruppe, eine (C₁-C₆)-Hydroxyalkyl-(C₁-C₆)-aminoalkylgruppe, eine (C₁-C₆)-Alkoxy-(C₁-C₆)-alkylgruppe, eine Carbonsäuregruppe oder ein gegebenenfalls substituiertes aromatisches (isozyklisches oder heterozyklisches) Ringsystem darstellen, wobei auch zwei benachbarte Reste **R1** bis **R4** gemeinsam einen ankondensierten aromatischen (isozyklischen oder heterozyklischen) Ring bilden können.

Als geeignete Verbindungen der Formel (I) können beispielweise genannt werden:
1,4-Benzochinon-4-[O-(methylsulfonyl)oxim],
2-Methyl-1,4-benzochinon-4-[O-(methylsulfonyl)oxim],
2-Chlor-1,4-benzochinon-4-[O-(methylsulfonyl)oxim],
2-Brom-1,4-benzochinon-4-[O-(methylsulfonyl)oxim],
2-Fluor-1,4-benzochinon-4-[O-(methylsulfonyl)oxim],
2-Hydroxymethyl-1,4-benzochinon-4-[O-(methylsulfonyl)oxim],
2-Hydroxyethyl-1,4-benzochinon-4-[O-(methylsulfonyl)oxim],
2-Aminomethyl-1,4-benzochinon-4-[O-(methylsulfonyl)oxim],
2-Methoxymethyl-1,4-benzochinon-4-[O-(methylsulfonyl)oxim],
2-[(2-Hydroxyethyl)-amino]methyl-1,4-benzochinon-4-[O-(methylsulfonyl)oxim],
2-Hydroxycarbonyl-1,4-benzochinon-4-[O-(methylsulfonyl)oxim],
3-Methyl-1,4-benzochinon-4-[O-(methylsulfonyl)oxim],
3-Chlor-1,4-benzochinon-4-[O-(methylsulfonyl)oxim],
3-Brom-1,4-benzochinon-4-[O-(methylsulfonyl)oxim],
3-Fluor-1,4-benzochinon-4-[O-(methylsulfonyl)oxim],
3-Hydroxymethyl-1,4-benzochinon-4-[O-(methylsulfonyl)oxim],
2,6-Dichlor-1,4-benzochinon-4-[O-(methylsulfonyl)oxim],
2,6-Dibrom-1,4-benzochinon-4-[O-(methylsulfonyl)oxim],
2,5-Dimethyl-1,4-benzochinon-4-[O-(methylsulfonyl)oxim],
2-Isopropyl-5-methyl-1,4-benzochinon-4-[O-(methylsulfonyl)oxim],
2-Methyl-5-isopropyl-1,4-benzochinon-4-[O-(methylsulfonyl)oxim],
1,4-Naphtochinon-4-[O-methylsulfonyl)oxim],
1-Phenylimin-benzochinon-4-[O-(methylsulfonyl)oxim],
1-Methylimin- benzochinon-4-[O-(methylsulfonyl)oxim],
1,4-Benzochinon-4-[O-(phenylsulfonyl)oxim],
2-Methyl-1,4-benzochinon-4-[O-(phenylsulfonyl)oxim],
2-Chlor-1,4-benzochinon-4-[O-(phenylsulfonyl)oxim],
2-Brom-1,4-benzochinon-4-[O-(phenylsulfonyl)oxim],
2-Fluor-1,4-benzochinon-4-[O-(phenylsulfonyl)oxim],
2-Hydroxymethyl-1,4-benzochinon-4-[O-(phenylsulfonyl)oxim],
2-Hydroxyethyl-1,4-benzochinon-4-[O-(phenylsulfonyl)oxim],
2-Aminomethyl-1,4-benzochinon-4-[O-(phenylsulfonyl)oxim],
2-Methoxymethyl-1,4-benzochinon-4-[O-(phenylsulfonyl)oxim],
2-[(2-Hydroxyethyl)-amino]methyl-1,4-benzochinon-4-[O-(phenylsulfonyl)oxim],
2-Hydroxycarbonyl-1,4-benzochinon-4-[O-(phenylsulfonyl)oxim],
3-Methyl-1,4-benzochinon-4-[O-(phenylsulfonyl)oxim],
3-Chlor-1,4-benzochinon-4-[O-(phenylsulfonyl)oxim],
3-Brom-1,4-benzochinon-4-[O-(phenylsulfonyl)oxim],
3-Fluor-1,4-benzochinon-4-[O-(phenylsulfonyl)oxim],
3-Hydroxymethyl-1,4-benzochinon-4-[O-(phenylsulfonyl)oxim],
2,6-Dichlor-1,4-benzochinon-4-[O-(phenylsulfonyl)oxim],
2,6-Dibrom-1,4-benzochinon-4-[O-(phenylsulfonyl)oxim],
1,4-Benzochinon-4-[O-4-(tolylsulfonyl)oxim],
2-Methyl-1,4-benzochinon-4-[O-4-(tolylsulfonyl)oxim],
2-Chlor-1,4-benzochinon-4-[O-4-(tolylsulfonyl)oxim],
2-Brom-1,4-benzochinon-4-[O-4-(tolylsulfonyl)oxim],
2-Fluor-1,4-benzochinon-4-[O-4-(tolylsulfonyl)oxim],
2-Hydroxymethyl-1,4-benzochinon-4-[O-4-(tolylsulfonyl)oxim],
2-Hydroxyethyl-1,4-benzochinon-4-[O-4-(tolylsulfonyl)oxim],
2-Aminomethyl-1,4-benzochinon-4-[O-4-(tolylsulfonyl)oxim],
2-Methoxymethyl-1,4-benzochinon-4-[O-4-(tolylsulfonyl)oxim],
2-[(2-Hydroxyethyl)-amino]methyl-1,4-benzochinon-4-[O-4-(tolylsulfonyl)oxim],
2-Hydroxycarbonyl-1,4-benzochinon-4-[O-4-(tolylsulfonyl)oxim],
3-Methyl-1,4-benzochinon-4-[O-4-(tolylsulfonyl)oxim],
3-Chlor-1,4-benzochinon-4-[O-4-(tolylsulfonyl)oxim],
3-Brom-1,4-benzochinon-4-[O-4-(tolylsulfonyl)oxim],
3-Fluor-1,4-benzochinon-4-[O-4-(tolylsulfonyl)oxim],
3-Hydroxymethyl-1,4-benzochinon-4-[O-4-(tolylsulfonyl)oxim],
2,6-Dichlor-1,4-benzochinon-4-[O-4-(tolylsulfonyl)oxim],
2,6-Dibrom-1,4-benzochinon-4-[O-4-(tolylsulfonyl)oxim],
1,4-Benzochinon-4-(O-acetyloxim),
2-Methyl-1,4-benzochinon-4-(O-acetyloxim),
2-Chlor-1,4-benzochinon-4-(O-acetyloxim),
2-Brom-1,4-benzochinon-4-(O-acetyloxim),
2-Fluor-1,4-benzochinon-4-(O-acetyloxim),
2-Hydroxymethyl-1,4-benzochinon-4-(O-acetyloxim),
2-Hydroxyethyl-1,4-benzochinon-4-(O-acetyloxim),
2-Aminomethyl-1,4-benzochinon-4-(O-acetyloxim),
2-Methoxymethyl-1,4-benzochinon-4-(O-acetyloxim),
2-[(2-Hydroxyethyl)-amino]methyl-1,4-benzochinon-4-(O-acetyloxim),
2-Hydroxycarbonyl-1,4-benzochinon-4-(O-acetyloxim),
3-Methyl-1,4-benzochinon-4-(O-acetyloxim),
3-Chlor-1,4-benzochinon-4-(O-acetyloxim),
3-Brom-1,4-benzochinon-4-(O-acetyloxim),
3-Fluor-1,4-benzochinon-4-(O-acetyloxim),
3-Hydroxymethyl-1,4-benzochinon-4-(O-acetyloxim),
2,6-Dichlor-1,4-benzochinon-4-(O-acetyloxim) und
2,6-Dibrom-1,4-benzochinon-4-(O-acetyloxim).

Unter den vorgenannten Verbindungen der Formel (I) sind die folgenden Verbindungen besonders bevorzugt:
1,4-Benzochinon-4-[O-(methylsulfonyl)oxim],
2-Methyl-1,4-benzochinon-4-[O-(methylsulfonyl)oxim],
2-Chlor-1,4-benzochinon-4-[O-(methylsulfonyl)oxim],
2-Brom-1,4-benzochinon-4-[O-(methylsulfonyl)oxim],
2-Fluor-1,4-benzochinon-4-[O-(methylsulfonyl)oxim],
2-Hydroxymethyl-1,4-benzochinon-4-[O-(methylsulfonyl)oxim],
2-Hydroxyethyl-1,4-benzochinon-4-[O-(methylsulfonyl)oxim],
2-Aminomethyl-1,4-benzochinon-4-[O-(methylsulfonyl)oxim],
2-Methoxymethyl-1,4-benzochinon-4-[O-(methylsulfonyl)oxim],
2-[(2-Hydroxyethyl)-amino]methyl-1,4-benzochinon-4-[O-(methylsulfonyl)oxim],
2-Hydroxycarbonyl-1,4-benzochinon-4-[O-(methylsulfonyl)oxim],
3-Methyl-1,4-benzochinon-4-[O-(methylsulfonyl)oxim],
3-Chlor-1,4-benzochinon-4-[O-(methylsulfonyl)oxim],
3-Brom-1,4-benzochinon-4-[O-(methylsulfonyl)oxim],
3-Fluor-1,4-benzochinon-4-[O-(methylsulfonyl)oxim],
3-Hydroxymethyl-1,4-benzochinon-4-[O-(methylsulfonyl)oxim],
2,6-Dichlor-1,4-benzochinon-4-[O-(methylsulfonyl)oxim],
2,6-Dibrom-1,4-benzochinon-4-[O-(methylsulfonyl)oxim],
2,5-Dimethyl-1,4-benzochinon-4-[O-(methylsulfonyl)oxim],
2-Isopropyl-5-methyl-1,4-benzochinon-4-[O-(methylsulfonyl)oxim],
2-Methyl-5-isopropyl-1,4-benzochinon-4-[O-(methylsulfonyl)oxim] und
1,4-Naphtochinon-4-[O-methylsulfonyl)oxim].

Als aromatische Amine kommen aromatische (isozyklische oder heterozyklische) Verbindungen mit mindestens einer Aminogruppe in Betracht, während als Phenole aromatische (isozyklische oder heterozyklische) Verbindungen mit mindestens einer Hydroxygruppe in Betracht kommen.
Als Beispiele für die in der Komponente (A2) enthaltenen Amine und Phenole können insbesondere genannt werden:
N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxy-propoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, Phenol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

Die Verbindungen der Formel (I) sowie die Amine und/oder Phenole werden getrennt voneinander aufbewahrt und erst kurz vor der Anwendung miteinander vermischt. Es ist jedoch auch möglich, sofern die Verbindungen der Formel (I) sowie die Amine und/oder Phenole in fester Form vorliegen, diese gemeinsam abzupacken und das gebrauchsfertige Färbemittel (A) kurz vor der Anwendung durch Vermischen der Verbindungen der Formel (I) und der Amine und/oder Phenole mit Wasser oder einer die übrigen Bestandteile des Mittels enthaltenden flüssigen Zubereitung herzustellen.

Weiterhin kann das erfindungsgemäße Färbemittel zusätzlich zu den Verbindungen der Formel (I) sowie den Aminen und Phenolen in der Komponente (A2) sowie der gebrauchsfertigen Zubereitung (A) gegebenenfalls zusätzlich weitere übliche, physiologisch unbedenkliche, direktziehende Farbstoffe aus der Gruppe der Nitrofabstoffe, der kationischen und anionischen Farbstoffe, der Dispersionsfarbstoffe, der Azofarbstoffe, der Chinonfarbstoffe und der Triphenylmethanfarbstoffe enthalten.

Diese direktziehenden Farbstoffe können in der Komponente (A2) in einer Gesamtmenge von etwa 0,02 bis 20 Gewichtsprozent, vorzugsweise 0,2 bis 10 Gewichtsprozent, eingesetzt werden, wobei die Gesamtmenge an direktziehenden Farbstoffen in dem durch Vermischen der Komponenten (A1) und (A2) erhaltenen gebrauchsfertigen Färbemittel (A) etwa 0,01 bis 10 Gewichtsprozent, vorzugsweise 0,1 bis 5 Gewichtsprozent, beträgt.

Das erfindungsgemäße Färbemittel besteht in der Regel aus einer Mischung der Komponenten (A1) und (A2), nämlich einer Farbträgermasse (A1), welche die Verbindung der Formel (I) enthält, und einer weiteren Farbträgermasse (A2), welche die Amine und/oder Phenole enthält.

Die Verbindungen der Formel (I) sowie die Amine und/oder Phenole sind in der jeweiligen Farbträgermasse (Komponente (A1) bzw. Komponente (A2)) jeweils in einer Gesamtmenge von etwa 0,02 bis 20 Gewichtsprozent, vorzugsweise etwa 0,2 bis 10 Gewichts-prozent, enthalten, wobei in dem gebrauchsfertigen Färbemittel (A) die Verbindungen der Formel (I) sowie die Amine und/oder Phenole jeweils in einer Gesamtmenge von etwa 0,01 bis 10 Gewichtsprozent, vorzugsweise etwa 0,1 bis 5 Gewichtsprozent, enthalten sind.

Die Zubereitungsform für die Komponenten (A1) und (A2) sowie des gebrauchsfertigen Färbemittels (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Verbindung der Formel (I) beziehungsweise der Amine und/oder Phenole mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylmmoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (jeweils bezogen auf die Komponente (A1) bzw. (A2)), die Verdicker in einer Menge von etwa 0,1 bis 25 Gewichtsprozent (jeweils bezogen auf die Komponente (A1) bzw. (A2)) und die Pflegestoffe in einer Konzentration von etwa 0,1 bis 5,0 Gewichtsprozent (jeweils bezogen auf die Komponente (A1) bzw. (A2)).

Der pH-Wert des gebrauchsfertigen Färbemittels (A) beträgt etwa 3 bis 12, vorzugsweise etwa 4 bis 10, und stellt sich in der Regel bei der Mischung der Komponente (A1) mit der Komponente (A2) ein.

Zur Einstellung des für die Färbung gewünschten pH-Wertes der Komponenten (A1) und (A2) sowie des gebrauchsfertigen Färbemittels (A) können -falls erforderlich- alkalisierende Mittel, wie zum Beispiel Alkalihydroxide, Erdalkalihydroxide, Alkaliacetate, Erdalkaliacetate, Alkalicarbonate oder Erdalkalicarbonate, oder Säuren, wie zum Beispiel Milchsäure, Essigsäure, Weinsäure, Phosphorsäure, Salzsäure, Zitronensäure, Ascorbinsäure oder Borsäure, verwendet werden.

Das gebrauchsfertige Färbemittel wird unmittelbar vor der Anwendung durch Vermischen der Komponenten (A1) und (A2) -gegebenenfalls unter Zusatz von Natriumcarbonat, Natriumhydrogencarbonat oder Natriumacetat- hergestellt und sodann auf die Faser, insbesondere menschliche Haare, aufgetragen. Je nach gewünschter Farbtiefe läßt man diese Mischung etwa 5 bis 60 Minuten, vorzugsweise etwa 15 bis 30 Minuten, bei einer Temperatur von etwa 20 bis 50 °C, insbesondere bei etwa 30 bis 40 °C einwirken. Anschließend wird die Faser mit Wasser gespült, gegebenenfalls mit einem Shampoo gewaschen und sodann getrocknet.

Ein weiterer Gegenstand der vorliegenden Erfindung ist ein Mehrkomponenten-Kit, bestehend aus einem Mittel der Komponente (A1), einem Mittel der Komponente (A2), sowie gegebenenfalls einem Mittel zur Einstellung des pH-Wertes. Selbstverständlich können auch die Mittel der Komponenten (A1) und (A2) aus mehreren Einzelkomponenten bestehen, welche erst unmittelbar vor der Anwendung miteinander vermischt werden. Ebenfalls ist ein 2-Komponenten-Kit möglich, dessen 1. Komponente aus einem die Verbindungen der Formel (I) sowie die Amine und/oder Phenole und gegebenenfalls weitere übliche pulverförmige kosmetische Zusatzstoffe enthaltenden Pulver besteht, und dessen 2. Komponente Wasser oder eine flüssige kosmetische Zubereitung ist. Besonders bevorzugt ist jedoch ein 2-Komponenten-Kit, bestehend aus einem Mittel der Komponente (A1) und einem Mittel der Komponente (A2).

Das erfindungsgemässe Färbemittel ermöglicht eine schonende, gleichmässige und dauerhafte Färbung der Fasern, insbesondere von Keratinfasern, wie zum Beispiel menschlichen Haaren, wobei eine breite Farbpalette von gelben bis braun-schwarzen Farbtönen möglich ist. Obwohl eine Verwendung des vorgenannten Mittels ohne den Zusatz von Oxidationsmitteln bevorzugt ist, ist eine Verwendung des vorgenannten Färbemittels in Verbindung mit Oxidationsmitteln ohne weiteres möglich, beispielsweise wenn eine gleichzeitige Bleichung der Faser gewünscht wird oder wenn dem Färbemittel auch übliche Oxidationsfarbstoffvorstufen zugesetzt werden sollen.

Die Verbindungen der Formel (I) können nach an sich bekannten Methoden hergestellt werden, beispielsweise in Analogie zu den in der DE-OS 12 03 042; den Analen der Chemie, 37 (1965), Seiten 1395-1399, oder dem Australian Journal Chemistry, 24 (1971), Seiten 1449-1465, beschriebenen Verfahren durch Umsetzung der entsprechenden, größtenteils literaturbekannten oder im Handel erhältlichen 1,4-Benzochinon-4-oxime der Formel (II) mit einem Sulfonsäurehalogenid oder Acetanhydrid.

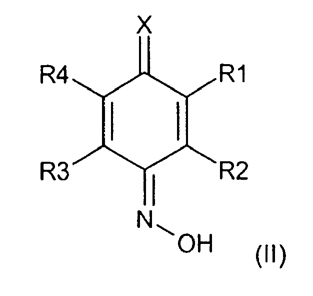

Die 1,4-Benzochinon-4-oxime der Formel (II) können beispielsweise in Analogie zu den in Journal of Organic Chemistry 61 (1996), Seiten 2774-2779, oder in Tetrahedron 51 (1995), Seiten 8447-8458 beschriebenen Verfahren hergestellt werden.

Die nachfolgenden Beispiele sollen den Gegenstand der Erfindung näher erläutern, ohne ihn auf diese Beispiele zu beschränken.

### Beispiele

### I. Herstellungsbeispiele

### Beispiel 1: Synthese von 3-Methyl-1,4-benzochinon-4-[O-(methylsulfonyl)oxim]

### A. Synthese von 3-Methyl-1,4-benzochinon-4-oxim

Das 3-Methyl-1,4-benzochinon-4-oxim wird nach der von T. Ishikawa *et al,* in Tetrahedron 1995, 31, Seite 8454 beschriebenen Methode durch Umsetzung von 3-Methylphenol mit Natriumnitrit unter basischen Bedingungen hergestellt.

### B. Synthese von 3-Methyl-1,4-benzochinon-4-[O-(methylsulfonyl)oxim]

Eine Lösung von 1 g (7,28 mmol) 3-Methyl-1,4-benzochinon-4-oxim in 10 ml Tetrahydrofuran und 0,7 g Triethylamin wird auf 0°C gekühlt. Das Reaktionsgemisch wird sodann bei 0 °C tropfenweise mit 0,92 g (8 mmol) Methansulfochlorid versetzt. Nach beendeter Reaktion wird das Reaktionsgemisch auf 150 ml Wasser/Eis gegossen. Das ausgefallene Produkt wird abfiltriert, mit Wasser gewaschen und sodann getrocknet.
Ausbeute: 1,28 g (82% der Theorie).
Schmelzpunkt: 135-136 °C
Massenspektrum (ESI-MS): 238 [M+Na]⁺ (100)
Elementaranalyse:

| C₈H₉NO₄S(215,23) | | | |
|---|---|---|---|
| | %C | %H | %N |
| Ber.: | 44,64 | 4,22 | 6,51 |
| Gef.: | 45,02 | 4,18 | 6,18 |

### Beispiel 2: Synthese von 2-Methyl-1,4-benzochinon-4-[O-(methylsulfonyl)oxim]

### A. Synthese von 2-Methyl-1,4-benzochinon-4-oxim

Das 2-Methyl-1,4-benzochinon-4-oxim wird nach der von von T. Ishikawa *et al,* in J. Org, Chem. 1996, 61, Seite 2778 beschriebenen Methode durch Umsetzung von 2-Methylphenol mit Natriumnitrit unter sauren Bedingungen hergestellt.

### B. Synthese von 2-Methyl-1,4-benzochinon-4-[O-(methylsulfonyl)oxim]

0,7 g (5,1 mmol) 2-Methyl-1,4-benzochinon-4-oxim werden in Analogie zur Vorschrift aus Beispiel **1B** umgesetzt. Es werden 0,9 g (85 % der Theorie) 2-Methyl-1,4-benzochinon-4-[O-(methylsulfonyl)oxim] erhalten.
Schmelzpunkt: 100-102 °C
Massenspektrum (ESI-MS): 238 [M+Na] ⁺ (100)

### Beispiel 3: Synthese von 3-Methyl-1,4-benzochinon-4-[O-(tolylsulfonyl)oxim]

Eine Lösung von 1 g (7,28 mmol) 3-Methyl-1,4-benzochinon-4-oxim in 10 ml Tetrahydrofuran und 0,7 g Triethylamin wird auf 0 °C gekühlt. Das Reaktionsgemisch wird sodann bei 0 °C tropfenweise mit 1,52 g (8 mmol) Toluol-4-sulfochlorid versetzt. Nach beendeter Reaktion wird das Reaktionsgemisch auf 200 ml Wasser/Eis gegossen. Das ausgefallene Produkt wird abfiltriert, mit Wasser gewaschen und sodann getrocknet.
Ausbeute: 1,8 g (85% der Theorie).
Schmelzpunkt: 98-100 °C
Massenspektrum (ESI-MS): 314 [M+Na]⁺ (100)
Elementaranalyse:

| C₁₄H₁₃NO₄S(291,32) | | | |
|---|---|---|---|
| | %C | %H | %N |
| Ber.: | 57,72 | 4,50 | 4,81 |
| Gef.: | 57,54 | 4,58 | 4,44 |

### Beispiel 4: Synthese von 2-Methyl-1,4-benzochinon-4-[O-(tolyl-sulfonyl)oxim]

1,0 g (7,3 mmol) 2-Methyl-1,4-benzochinon-4-oxim werden in Analogie zur Vorschrift aus Beispiel 3 umgesetzt. Es werden 1,87 g (88 % der Theorie) 2-Methyl-1,4-benzochinon-4-[O-(tolylsulfonyl)oxim] erhalten.
Schmelzpunkt: 138-140°C
Massenspektrum (ESI-MS): 314 [M+Na]⁺ (100)

### Beispiel 5: Synthese von 2-Chlor-1,4-benzochinon-4-[O-(tolyl-sulfonyl)oxim]

### A. Synthese von 2-Chlor-1,4-benzochinon-4-oxim

Das 2-Chlor-1,4-benzochinon-4-oxim wird gemäß der von T. Ishikawa *et al*, in J. Org, Chem. 1996, 61, Seite 2778 beschriebenen Methode durch Umsetzung von 2-Chlorphenol mit Natriumnitrit unter sauren Bedingungen hergestellt.

### B. Synthese von 2-Chlor-1,4-benzochinon-4-[O-(tolylsulfonyl)oxim]

0,5 g (3,2 mmol) 2-Chlor-1,4-benzochinon-4-oxim werden in Analogie zur Vorschrift aus Beispiel 3 umgesetzt. Es werden 0,8 g (82 % der Theorie) 2-Chlor-1,4-benzochinon-4-[O-(tolylsulfonyl)oxim] erhalten.
Schmelzpunkt: 164-166°C
Massenspektrum (ESI-MS): 334 [M+Na]+ (100)

### Beispiel 6: Synthese von 3-Methyl-1,4-benzochinon-4-(O-acetyloxim)

Eine Mischung von 0,5 g (3,6 mmol) 3-Methyl-1,4-benzochinon-4-oxim in 1 ml Essigsäureanhydrid wird mit 1 Tropfen Pyridin versetzt und auf 70°C erwärmt. Nach beendeter Reaktion wird das Reaktionsgemisch auf 10 ml Wasser/Eis gegossen. Das ausgefallene Produkt wird abfiltriert, mit Wasser gewaschen und sodann getrocknet.
Ausbeute: 0,32 g (50 % der Theorie).
Schmelzpunkt: 84-87 °C
Massenspektrum (ESI-MS): 202 [M+Na]⁺ (100)
Elementaranalyse:

| C₉H₉NO₃ (179,18) | | | |
|---|---|---|---|
| | %C | %H | %N |
| Ber.: | 60,33 | 5,06 | 7,82 |
| Gef.: | 60,62 | 5,03 | 7,47 |

### II. Beispiele für Färbemittel

### Beispiele 7 bis 28: Haarfärbemittel

Es werden Haarfärbelösungen der folgenden Zusammensetzung hergestellt:

### Komponente (A1)

| | |
|---|---|
| 0,00625 mol | Cinoniminderivat der Formel (I) gemäß Tabelle 1 |
| 5,0 g | Ethanol |
| 4,0 g | Decylpolyglucosid (wässrige Lösung; Plantaren® 2000 der Firma Cognis, Deutschland) |
| 0,2 g | Ethylendiaminotetraessigsäure-Dinatriumsalz-Hydrat |
| ad 100,0 g | Wasser, vollentsalzt |

### Komponente (A2)

| | |
|---|---|
| 0.00625 mol | Amin und/oder Phenol gemäß Tabelle 1 (in Pulverform) |

Bei Raumtemperatur (20-25 °C) oder unter leichtem Erwärmen (35-40 °C) wird die vorstehend genannte Mischung (Komponente (A1)) mit dem das Amin und/oder Phenol enthaltenden Pulver (Komponente (A2)) unter Zusatz einigen Tropfen einer gesättigten NatriumhydrogencarbonatLösung homogen vermischt.
Die pH-Werte werden -falls erforderlich- mit Natronlauge oder Zitronensäure auf den gewünschten Wert eingestellt.

Das so erhaltene gebrauchsfertige Haarfärbemittel wird auf das Haar aufgetragen und mit einem Pinsel gleichmäßig verteilt. Nach einer Einwirkungszeit von 30 Minuten bei 40 °C wird das Haar mit lauwarmem Wasser gespült und sodann getrocknet.

Die erhaltenen Färbungen sind in der nachfolgenden Tabelle 1 zusammengefasst.

**Tabelle 1:**

| **Beispiel** | **Komponent A1 : Verbindung der Formel (I)** | **Komponente A2 : Amin bzw. Phenol** | **erhaltene Färbung** | **Intensität** |
|---|---|---|---|---|
| **7** | 3-Methyl-1,4-benzochinon-4-[O-(methylsulfonyl)oxim] | 3-Aminophenol | dunkelblond | ++ |
| **8** | 3-Methyl-1,4-benzochinon-4-[O-(methylsulfonyl)oxim] | 1,3-Dihydroxybenzol | aschblond | + |
| **9** | 3-Methyl-1,4-benzochinon-4-[O-(methylsulfonyl)oxim] | 5-Amino-2-methylphenol | rot | +++ |
| **10** | 3-Methyl-1,4-benzochinon-4-[O-(methylsulfonyl)oxim] | 2-Amino-4-[(2-hydroxyethyl)-amino]-anisol | violett | +++ |
| **11** | 2-Methyl-1,4-benzochinon-4-[O-(methylsulfonyl)oxim] | 3-Aminophenol | dunkelblond | ++ |
| **12** | 2-Methyl-1,4-benzochinon-4-[O-(methylsulfonyl)oxim] | 1,3-Dihydroxybenzol | aschblond | + |
| **13** | 2-Methyl-1,4-benzochinon-4-[O-(methylsulfonyl)oxim] | 5-Amino-2-methylphenol | orange-rot | +++ |
| **14** | 2-Methyl-1,4-benzochinon-4-[O-(methylsulfonyl)oxim] | 2-Amino-4-[(2-hydroxyethyl)-amino]-anisol | braun-violett | +++ |
| **15** | 3-Methyl-1,4-benzochinon-4-[O-(tolylsulfonyl)oxim] | 3-Aminophenol | blond | + |
| **16** | 3-Methyl-1,4-benzochinon-4-[O-(tolylsulfonyl)oxim] | 1,3-Dihydroxybenzol | aschblond | + |
| **17** | 3-Methyl-1,4-benzochinon-4-[O-(tolylsulfonyl)oxim] | 5-Amino-2-methylphenol | rot | ++ |
| **18** | 3-Methyl-1,4-benzochinon-4-[O-(tolylsulfonyl)oxim] | 2-Amino-4-[(2-hydroxyethyl)-amino]-anisol | violett | ++ |
| **19** | 2-Methyl-1,4-benzochinon-4-[O-(tolylsulfonyl)oxim] | 3-Aminophenol | dunkelblond | ++ |
| **20** | 2-Methyl-1,4-benzochinon-4-[O-(tolylsulfonyl)oxim] | 1,3-Dihydroxybenzol | aschblond | + |
| **21** | 2-Methyl-1,4-benzochinon-4-[O-(tolylsulfonyl)oxim] | 5-Amino-2-methylphenol | orange-rot | ++ |
| **22** | 2-Methyl-1,4-benzochinon-4-[O-(tolylsulfonyl)oxim] | 2-Amino-4-[(2-hydroxyethyl)-amino]-anisol | braun-violett | ++ |
| **23** | 2-Chlor-1,4-benzochinon-4-[O-(tolylsulfonyl)oxim] | 3-Aminophenol | dunkelblond | ++ |
| **24** | 2-Chlor-1,4-benzochinon-4-[O-(tolylsulfonyl)oxim] | 1,3-Dihydroxybenzol | blond | + |
| **25** | 2-Chlor-1,4-benzochinon-4-[O-(tolylsulfonyl)oxim] | 5-Amino-2-methylphenol | braun-rot | ++ |
| **26** | 2-Chlor-1,4-benzochinon-4-[O-(tolylsulfonyl)oxim] | 2-Amino-4-[(2-hydroxyethyl)-amino]-anisol | violett | +++ |
| **27** | 3-Methyl-1,4-benzochinon-4-(O-acetyloxim) | 5-Amino-2-methyl-phenol | gelb | ++ |
| **28** | 3-Methyl-1,4-benzochinon-4-(O-acetyloxim) | 2-Amino-4-[(2-hydroxyethyl)-amino]-anisol | braun-violett | ++ |

| | | | | |
|---|---|---|---|---|
| +: schwache Intensität; ++: mittlere Intensität; +++: starke Intensität | | | | |

Alle Prozentangaben stellen soweit nicht anders angegeben Gewichtsprozente dar.

## Patentansprüche

1. Mittel zur Färbung von Fasern (A), welches durch Vermischen zweier Komponenten (A1) und (A2) hergestellt wird, **dadurch gekennzeichnet, dass** die Komponente (A1) mindestens ein Chinoniminderivat der Formel (I) enthält, und die Komponente (A2) mindestens eine Verbindung aus der Gruppe bestehend aus aromatischen Aminen und Phenolen enthält; 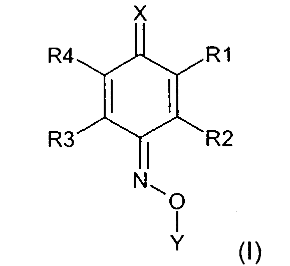 wobei in Formel (I)
**X** gleich Sauerstoff oder einer NR-Gruppe ist, mit R gleich einer gegebenenfalls substituierten (C₁-C₆)-Alkylgruppe oder einem gegebenenfalls substituierten aromatischen -isozyklischen oder heterozyklischen- Ring ;
**Y** gleich einer (C₁-C₆)-Alkylsulfonylgruppe, einer Arylsulfonyl oder einer Acetylgruppe ist;
**R1, R2, R3** und **R4** gleich oder verschieden sein können und unabhängig voneinander Wasserstoff, ein Halogenatom, eine (C₁-C₆)-Alkylgruppe, eine Trifluormethylgruppe, eine mit einem Halogenatom substituierte (C₁-C₆)-Alkylgruppe, eine (C₁-C₆)-Hydroxyalkyl-gruppe, eine (C₂-C₆)-Polyhydroxyalkylgruppe, eine (C₁-C₆)-Aminoalkyl-gruppe, eine (C₁-C₆)-Alkoxygruppe, eine (C₁-C₆)-Hydroxyalkoxygruppe, eine (C₁-C₆)-Aminoalkoxygruppe, eine (C₁-C₆)-Alkoxy-(C₁-C₆)-alkylgruppe, eine (C₁-C₆)-Alkoxy-(C₁-C₆)-alkoxygruppe, eine (C₁-C₆)-Hydroxyakyl-(C₁-C₆)-aminoalkylgruppe, eine Nitrogruppe, eine Cyanogruppe, eine Carbonsäuregruppe, eine Carbonsäureestergruppe oder ein gegebenenfalls substituiertes aromatisches (-isozyklisches oder heterozyklisches) Ringsystem darstellen, wobei auch zwei benachbarte Reste **R1** bis **R4** gemeinsam ein ankondensiertes aromatisches Ringsystem, das isozyklisch oder heterozyklischut, bilden können.

2. Mittel nach Anspruch 1, **dadurch gekennzeichnet, dass** in der Formel (I) **X** gleich Sauerstoff ist, **Y** gleich einer Methylsulfonylgruppe, einer Phenylsulfonylgruppe, einer 4-Tolylsulfonylgruppe, einer 4-Chlorphenylsulfonylgruppe, einer Acetylgruppe oder Chloracetylgruppe ist, und **R1, R2, R3** und **R4** gleich oder verschieden sein können und unabhängig voneinander Wasserstoff, ein Halogenatom, eine (C₁-C₆)-Alkylgruppe, eine (C₁-C₆)-Hydroxyalkylgruppe, eine (C₁-C₆)-Aminoalkylgruppe, eine (C₁-C₆)-Alkoxygruppe, eine (C₁-C₆)-Hydroxyalkyl-(C₁-C₆)-aminoalkylgruppe, eine (C₁-C₆)-Alkoxy-(C₁-C₆)-alkylgruppe, eine Carbonsäuregruppe oder ein gegebenenfalls substituiertes aromatisches (isozyklisches oder heterozyklisches) Ringsystem darstellen, wobei auch zwei benachbarte Reste **R1** bis **R4** gemeinsam einen ankondensierten aromatischen (isozyklischen oder heterozyklischen) Ring bilden können.

3. Mittel nach Anspruch 1, **dadurch gekennzeichnet, dass** das Chinoniminderivat der Formel (I) ausgewählt ist aus 1,4-Benzochinon-4-[O-(methylsulfonyl)oxim], 2-Methyl-1,4-benzochinon-4-[O-(methylsulfonyl)-oxim], 2-Chlor-1,4-benzochinon-4-[O-(methylsulfonyl)oxim], 2-Brom-1,4-benzochinon-4-[O-(methylsulfonyl)oxim], 2-Fluor-1,4-benzochinon-4-[O-(methylsulfonyl)oxim], 2-Hydroxymethyl-1,4-benzochinon-4-[O-(methylsulfonyl)oxim], 2-Hydroxyethyl-1,4-benzochinon-4-[O-(methylsulfonyl)-oxim], 2-Aminomethyl-1,4-benzochinon-4-[O-(methylsulfonyl)oxim], 2-Methoxymethyl-1,4-benzochinon-4-[O-(methylsulfonyl)oxim], 2-[(2-Hydroxyethyl)-amino]methyl-1,4-benzochinon-4-[O-(methylsulfonyl)oxim], 2-Hydroxycarbonyl-1,4-benzochinon-4-[O-(methylsulfonyl)-oxim], 3-Methyl-1,4-benzochinon-4-[O-(methylsulfonyl)oxim], 3-Chlor-1,4-benzochinon-4-[O-(methylsulfonyl)oxim], 3-Brom-1,4-benzochinon-4-[O-(methyisulfonyl)oxim], 3-Fluor-1,4-benzochinon-4-[O-(methylsulfonyl)-oxim], 3-Hydroxymethyl-1,4-benzochinon-4-[O-(methylsulfonyl)oxim], 2,6-Dichlor-1,4-benzochinon-4-[O-(methylsulfonyl)oxim], 2,6-Dibrom-1,4-benzochinon-4-[O-(methylsulfonyl)oxim],2,5-Dimethyl-1,4-benzochinon-4-[O-(methylsulfonyl)oxim], 2-Isopropyl-5-methyl-1,4-benzochinon-4-[O-(methylsulfonyl)oxim], 2-Methyl-5-isopropyl-1,4-benzochinon-4-[O-(methylsulfonyl)oxim], 1,4-Naphtochinon-4-[O-methylsulfonyl)oxim], 1-Phenylimin-benzochinon-4-[O-(methylsulfonyl)oxim], 1-Methylimin- benzochinon-4-[O-(methylsulfonyl)oxim], 1,4-Benzochinon-4-[O-(phenylsulfonyl)oxim], 2-Methyl-1,4-benzochinon-4-[O-(phenylsulfonyl)oxim], 2-Chlor-1,4-benzochinon-4-[O-(phenylsulfonyl)oxim], 2-Brom-1,4-benzochinon-4-[O-(phenylsulfonyl)oxim], 2-Fluor-1,4-benzochinon-4-[O-(phenylsulfonyl)-oxim], 2-Hydroxymethyl-1,4-benzochinon-4-[O-(phenylsulfonyl)oxim], 2-Hydroxyethyl-1,4-benzochinon-4-[O-(phenylsulfonyl)oxim], 2-Aminomethyl-1,4-benzochinon-4-[O-(phenylsulfonyl)oxim], 2-Methoxymethyl-1,4-benzochinon-4-[O-(phenylsulfonyl)oxim], 2-[(2-Hydroxyethyl)-amino]methyl-1,4-benzochinon-4-[O-(phenylsulfonyl)-oxim], 2-Hydroxycarbonyl-1,4-benzochinon-4-[O-(phenylsulfonyl)oxim], 3-Methyl-1,4-benzochinon-4-[O-(phenylsulfonyl)oxim], 3-Chlor-1,4-benzochinon-4-[O-(phenylsulfonyl)oxim], 3-Brom-1,4-benzochinon-4-[O-(phenylsulfonyl)oxim], 3-Fluor-1,4-benzochinon-4-[O-(phenylsulfonyl)-oxim], 3-Hydroxymethyl-1,4-benzochinon-4-[O-(phenylsulfonyl)oxim], 2,6-Dichlor-1,4-benzochinon-4-[O-(phenylsulfonyl)oxim], 2,6-Dibrom-1,4-benzochinon-4-[O-(phenylsulfonyl)oxim], 1,4-Benzochinon-4-[O-4-(tolyl-sulfonyl)oxim], 2-Methyl-1,4-benzochinon-4-[O-4-(tolylsulfonyl)oxim], 2-Chlor-1,4-benzochinon-4-[O-4-(tolylsulfonyl)oxim], 2-Brom-1,4-benzochinon-4-[O-4-(tolylsulfonyl)oxim], 2-Fluor-1,4-benzochinon-4-[O-4-(tolylsulfonyl)oxim], 2-Hydroxymethyl-1,4-benzochinon-4-[O-4-(tolylsulfonyl)oxim], 2-Hydroxyethyl-1,4-benzochinon-4-[O-4-(tolylsulfonyl)-oxim], 2-Aminomethyl-1,4-benzochinon-4-[O-4-(tolylsulfonyl)oxim], 2-Methoxymethyl-1,4-benzochinon-4-[O-4-(tolylsulfonyl)oxim], 2-[(2-Hydroxyethyl)-amino]methyl-1,4-benzochinon-4-[O-4-(tolylsulfonyl)oxim], 2-Hydroxycarbonyl-1,4-benzochinon-4-[O-4-(tolylsulfonyl)oxim], 3-Methyl-1,4-benzochinon-4-[O-4-(tofylsulfonyl)oxim], 3-Chlor-1,4-benzochinon-4-[O-4-(tolylsulfonyl)oxim], 3-Brom-1,4-benzochinon-4-[O-4-(tolylsulfonyl)oxim], 3-Fluor-1,4-benzochinon-4-[O-4-(tolylsulfonyl)oxim], 3-Hydroxymethyl-1,4-benzochinon-4-[O-4-(tolylsulfonyl)oxim], 2,6-Dichlor-1,4-benzochinon-4-[O-4-(tolylsulfonyl)oxim], 2,6-Dibrom-1,4-benzochinon-4-[O-4-(tolylsulfonyl)oxim], 1,4-Benzochinon-4-(O-acetyloxim), 2-Methyl-1,4-benzochinon-4-(O-acetyloxim), 2-Chlor-1,4-benzochinon-4-(O-acetyloxim), 2-Brom-1,4-benzochinon-4-(O-acetyloxim), 2-Fluor-1,4-benzochinon-4-(O-acetyloxim), 2-Hydroxymethyl-1,4-benzochinon-4-(O-acetyloxim), 2-Hydroxyethyl-1,4-benzochinon-4-(O-acetyloxim), 2-Aminomethyl-1,4-benzochinon-4-(O-acetyloxim), 2-Methoxymethyl-1,4-benzochinon-4-(O-acetyloxim), 2-[(2-Hydroxyethyl)-amino]methyl-1,4-benzochinon-4-(O-acetyloxim), 2-Hydroxycarbonyl-1,4-benzochinon-4-(O-acetyloxim), 3-Methyl-1,4-benzochinon-4-(O-acetyloxim), 3-Chlor-1,4-benzochinon-4-(O-acetyloxim), 3-Brom-1,4-benzochinon-4-(O-acetyloxim), 3-Fluor-1,4-benzochinon-4-(O-acetyloxim), 3-Hydroxymethyl-1,4-benzochinon-4-(O-acetyloxim), 2,6-Dichlor-1,4-benzochinon-4-(O-acetyloxim) und 2,6-Dibrom-1,4-benzochinon-4-(O-acetyloxim).

4. Mittel nach Anspruch 1, **dadurch gekennzeichnet**, das das Chinoniminderivat der Formel (I) ausgewählt ist aus1,4-Benzochinon-4-[O-(methylsulfonyl)oxim], 2-Methyl-1,4-benzochinon-4-[O-(methylsulfonyl)-oxim], 2-Chlor-1,4-benzochinon-4-[O-(methylsulfonyl)oxim], 2-Brom-1,4-benzochinon-4-[O-(methylsulfonyl)oxim], 2-Fluor-1,4-benzochinon-4-[O-(methylsulfonyl)oxim], 2-Hydroxymethyl-1,4-benzochinon-4-[O-(methylsulfonyl)oxim], 2-Hydroxyethyl-1,4-benzochinon-4-[O-(methylsulfonyl)-oxim], 2-Aminomethyl-1,4-benzochinon-4-[O-(methylsulfonyl)oxim], 2-Methoxymethyl-1,4-benzochinon-4-[O-(methylsulfonyl)oxim], 2-[(2-Hydroxyethyl)-amino]methyl-1,4-benzochinon-4-[O-(methylsulfonyl)-oxim], 2-Hydroxycarbonyl-1,4-benzochinon-4-[O-(methylsulfonyl)oxim], 3-Methyl-1,4-benzochinon-4-[O-(methylsulfonyl)oxim], 3-Chlor-1,4-benzochinon-4-[O-(methylsulfonyl)oxim], 3-Brom-1,4-benzochinon-4-[O-(methylsulfonyl)oxim], 3-Fluor-1,4-benzochinon-4-[O-(methylsulfonyl)-oxim], 3-Hydroxymethyl-1,4-benzochinon-4-[O-(methylsulfonyl)oxim], 2,6-Dichlor-1,4-benzochinon-4-[O-(methylsulfonyl)oxim], 2,6-Dibrom-1,4-benzochinon-4-[O-(methylsulfonyl)oxim], 2,5-Dimethyl-1,4-benzochinon-4-[O-(methylsulfonyl)oxim], 2-Isopropyl-5-methyl-1,4-benzochinon-4-[O-(methylsulfonyl)oxim], 2-Methyl-5-isopropyl-1,4-benzochinon-4-[O-(methylsulfonyl)oxim] und 1,4-Naphtochinon-4-[O-methylsulfonyl)oxim].

5. Mittel nach einem der Ansprüche 1 bis 4, **dadurch gekennzeichnet, dass** das Amin oder Phenol ausgewählt ist aus N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 1,3-Diamino-4-(3-hydroxypropoxy)-benzol, 1,3-Diamino-4-(2-methoxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, Phenol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

6. Mittel nach einem der Ansprüche 1 bis 5, **dadurch gekennzeichnet, dass** es die Chinoniminderivate der Formel (I) sowie die Amine, und/oder Phenole in der jeweiligen Farbträgermasse (Komponente (A1) bzw. Komponente (A2)) jeweils in einer Gesamtmenge von 0,02 bis 20 Gewichtsprozent enthält.

7. Mittel nach einem der Ansprüche 1 bis 6, **dadurch gekennzeichnet, dass** es die Chinoniminderivate der Formel (I) sowie die Amine und/oder Phenole in dem gebrauchsfertigen Färbemittel (A) jeweils in einer Gesamtmenge von 0,01 bis 10 Gewichtsprozent enthält.

8. Mittel nach einem der Ansprüche 1 bis 7, **dadurch gekennzeichnet, dass** es zusätzlich 0,02 bis 20 Gewichtsprozent eines direktziehenden Farbstoffs aus der Gruppe der kationischen und anionischen Farbstoffe, der Dispersionsfarbstoffe, der Nitrofarbstoffe, der Azofarbstoffe, der Chinonfarbstoffe und der Triphenylmethanfarbstoffe enthält.

9. Mittel nach einem der Ansprüche 1 bis 8, **dadurch gekennzeichnet, dass** das gebrauchsfertige Färbemittel (A) einen pH-Wert von 3 bis 12 aufweist.

10. Mittel nach einem der Ansprüche 1 bis 9, **dadurch gekennzeichnet, dass** es ein Haarfärbemittel ist.

11. Gebrauchsfertiges Mittel zur Färbung von Fasern (A), **dadurch gekennzeichnet, dass** mindestens ein Chinoniminderivat der Formel (I) und mindestens eine Verbindung aus der Gruppe bestehend aus aromatischen Aminen und Phenolen enthält und einen pH-Wert von 3 bis 12 aufweist; 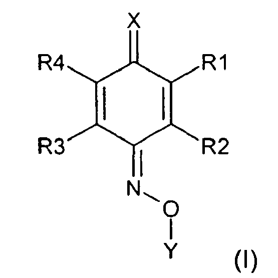 wobei in Formel (I)
**X** gleich Sauerstoff oder einer NR-Gruppe ist, mit R gleich einer gegebenenfalls substituierten (C₁-C₆)-Alkylgruppe oder einem gegebenenfalls substituierten aromatischen -isozyklischen oder heterozyklischen- Ring;
**Y** gleich einer (C₁-C₆)-Alkylsulfonylgruppe, einer Arylsulfonyl oder einer Acetylgruppe ist;
**R1, R2, R3** und **R4** gleich oder verschieden sein können und unabhängig voneinander Wasserstoff, ein Halogenatom, eine (C₁-C₆)-Alkylgruppe, eine Trifluormethylgruppe, eine mit einem Halogenatom substituierte (C₁-C₆)-Alkylgruppe, eine (C₁-C₆)-Hydroxyalkyl-gruppe, eine (C₂-C₆)-Polyhydroxyalkylgruppe, eine (C₁-C₆)-Aminoalkyl-gruppe, eine (C₁-C₆)-Alkoxygruppe, eine (C₁-C₆)-Hydroxyalkoxygruppe, eine (C₁-C₆)-Aminoalkoxygruppe, eine (C₁-C₆)-Alkoxy-(C₁-C₆)-alkylgruppe, eine (C₁-C₆)-Alkoxy-(C₁-C₆)-alkoxygruppe, eine (C₁-C₆)-Hydroxyakyl-(C₁-C₆)-aminoalkylgruppe, eine Nitrogruppe, eine Cyanogruppe, eine Carbonsäuregruppe, eine Carbonsäureestergruppe oder ein gegebenenfalls substituiertes aromatisches Ringsystem das isozyklisch oder heterozyklisch ist, darstellen, wobei auch zwei benachbarte Reste **R1** bis **R4** gemeinsam einen ankondensiertes aromatisches (isozyklisches oder heterozyklisches) Ringsystem bilden können.

12. Verfahren zum Färben von Keratinfasern bei dem das gebrauchsfertige Färbemittel (A) unmittelbar vor der Anwendung durch Vermischen zweier Komponenten (A1) und (A2) hergestellt und sodann auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird, **dadurch gekennzeichnet, dass** ein Färbemittel (A) nach einem der Ansprüche 1 bis 10 verwendet wird.

13. Mehrkomponenten-Kit zum Färben von Haaren, bestehend aus einem Mittel der Komponente (A1) gemäß einem der Ansprüche 1 bis 9 und einem Mittel der Komponente (A2) gemäß einem der Ansprüche 1 bis 9, sowie gegebenenfalls einem Mittel zur Einstellung des pH-Wertes.

## Claims

1. Agent for colouring fibres (A) which is prepared by mixing two components (A1) and (A2), **characterized in that** component (A1) comprises at least one quinonimine derivative of the formula (I), and component (A2) comprises at least one compound from the group consisting of aromatic amines and phenols; 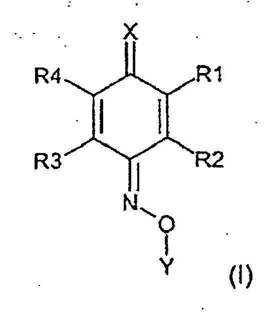 where, in formula (I),
X is oxygen or an NR group, where R is an optionally substituted (C₁-C₆)-alkyl group or an optionally substituted aromatic isocyclic or heterocyclic ring;
**Y** is a (C₁-C₆)-alkylsulphonyl group, an arylsulphonyl or an acetyl group;
**R1, R2, R3** and **R4** may be identical or different and, independently of one another, are hydrogen, a halogen atom, a (C₁-C₆)-alkyl group, a trifluoromethyl group, a (C₁-C₆)-alkyl group substituted by a halogen atom, a (C₁-C₆) -hydroxyalkyl group, a (C₂-C₆)-polyhydroxyalkyl group, a (C₁-C₆)-aminoalkyl group, a (C₁-C₆)-alkoxy group, a (C₁-C₆) -hydroxyalkoxy group, a (C₁-C₆)-aminoalkoxy group, a (C₁-C₆)-alkoxy- (C₁-C₆)-alkyl group, a (C₁-C₆)-alkoxy-(C₁-C₆)-alkoxy group, a (C₁-C₆)-hydroxyalkyl-(C₁-C₆)-aminoalkyl group, a nitro group, a cyano group, a carboxylic acid group, a carboxylic acid ester group or an optionally substituted aromatic (isocyclic or heterocyclic) ring system where two adjacent radicals **R1** to **R4** can together also form a fused-on aromatic ring system which is isocyclic or heterocyclic.

2. Agent according to Claim 1, **characterized in that**, in the formula (I), **X** is oxygen, **Y** is a methylsulphonyl group, a phenylsulphonyl group, a 4-tolylsulphonyl group, a 4-chlorophenylsulphonyl group, an acetyl group or chloroacetyl group, and **R1, R2, R3** and **R4** may be identical or different and, independently of one another, are hydrogen, a halogen atom, a (C₁-C₆)-alkyl group, a (C₁-C₆)-hydroxyalkyl group, a (C₁-C₆)-aminoalkyl group, a (C₁-C₆)-alkoxy group, a (C₁-C₆) -hydroxyalkyl-(C₁-C₆)-aminoalkyl group, a (C₁-C₆)-alkoxy-(C₁-C₆)-alkyl group, a carboxylic acid group or an optionally substituted aromatic (isocyclic or heterocyclic) ring system, where two adjacent radicals **R1** to **R4** can together also form a fused-on aromatic (isocyclic or heterocyclic) ring.

3. Agent according to Claim 1, **characterized in that** the quinonimine derivative of the formula (I) is chosen from 1,4-benzoquinone 4-[O-(methylsulphonyl)oxime], 2-methyl-1,4-benzoquinone 4-[O-(methylsulphonyl)oxime], 2-chloro-1,4-benzoquinone 4-[O-(methylsulphonyl)oxime], 2-bromo-1,4-benzoquinone 4-[O-(methylsulphonyl)oxime], 2-fluoro-1,4-benzoquinone 4-[O-(methylsulphonyl)oxime], 2-hydroxymethyl-1,4-benzoquinone 4-[O-(methylsulphonyl)oxime], 2-hydroxyethyl-1,4-benzoquinone 4-[O-(methylsulphonyl)oxime], 2-aminomethyl-1,4-benzoquinone 4-[O-(methylsulphonyl)oxime], 2-methoxymethyl-1,4-benzoquinone 4-[O-(methylsulphonyl)oxime], 2-[(2-hydroxyethyl)amino]methyl-1,4-benzoquinone 4-[O-(methylsulphonyl)oxime], 2-hydroxycarbonyl-1,4-benzoquinone 4-[O-(methylsulphonyl)oxime], 3-methyl-1,4-benzoquinone 4-[O-(methylsulphonyl)oxime], 3-chloro-1,4-benzoquinone 4-[O-(methylsulphonyl)oxime], 3-bromo-1,4-benzoquinone 4-[O-(methylsulphonyl)oxime], 3-fluoro-1,4-benzoquinone 4-[O-(methylsulphonyl)oxime], 3-hydroxymethyl-1,4-benzoquinone 4-[O-(methylsulphonyl)oxime], 2,6-dichloro-1,4-benzoquinone 4-[O-(methylsulphonyl)oxime], 2,6-dibromo-1,4-benzoquinone 4-[O-(methylsulphonyl)oxime], 2,5-dimethyl-1,4-benzoquinone 4-[O-(methylsulphonyl)oxime], 2-isopropyl-5-methyl-1,4-benzoquinone 4-[O-(methylsulphonyl)oxime], 2-methyl-5-isopropyl-1,4-benzoquinone 4-[O-(methylsulphonyl)oxime], 1,4-naphthoquinone 4-[O-(methylsulphonyl)oxime], 1-phenyliminobenzoquinone 4-[O-(methylsulphonyl)oxime], 1-methyliminobenzoquinone 4-[O-(methylsulphonyl)oxime], 1,4-benzoquinone 4-[O-(phenylsulphonyl)oxime], 2-methyl-1,4-benzoquinone 4-[O-(phenylsulphonyl)oxime], 2-chloro-1,4-benzoquinone 4-[O-(phenylsulphonyl)oxime], 2-bromo-1,4-benzoquinone 4-[O-(phenylsulphonyl)oxime], 2-fluoro-1,4-benzoquinone 4-[O-(phenylsulphonyl)oxime], 2-hydroxymethyl-1,4-benzoquinone 4-[O-(phenylsulphonyl)oxime], 2-hydroxyethyl-1,4-benzoquinone 4-[O-(phenylsulphonyl)oxime], 2-aminomethyl-1,4-benzoquinone 4-[O-(phenylsulphonyl)oxime], 2-methoxymethyl-1,4-benzoquinone 4-[O-(phenylsulphonyl)oxime], 2-[(2-hydroxyethyl)-amino]methyl-1,4-benzoquinone 4-[O-(phenylsulphonyl)-oxime], 2-hydroxycarbonyl-1,4-benzoquinone 4-[O-(phenylsulphonyl)oxime], 3-methyl-1,4-benzoquinone 4-[O-(phenylsulphonyl)oxime], 3-chloro-1,4-benzoquinone 4-[O-(phenylsulphonyl)oxime], 3-bromo-1,4-benzoquinone 4-[O-(phenylsulphonyl)oxime], 3-fluoro-1,4-benzoquinone 4-[O-(phenylsulphonyl)oxime], 3-hydroxymethyl-1,4-benzoquinone 4- [O- (phenylsulphonyl) oxime], 2,6-di-chloro-1,4-benzoquinone 4-[O-(phenylsulphonyl)oxime], 2,6-dibromo-1,4-benzoquinone 4-[O-(phenylsulphonyl)-oxime], 1,4-benzoquinone 4-[O-4-(tolylsulphonyl)oxime], 2-methyl-1,4-benzoquinone 4-[O-4-(tolylsulphonyl)oxime], 2-chloro-1,4-benzoquinone 4-[O-4-(tolylsulphonyl)oxime], 2-bromo-1,4-benzoquinone 4-[O-4-(tolylsulphonyl)oxime], 2-fluoro-1,4-benzoquinone 4-[O-4-(tolylsulphonyl)oxime], 2-hydroxymethyl-1,4-benzoquinone 4-[O-(tolylsulphonyl)oxime], 2-hydroxyethyl-1,4-benzoquinone 4-[O-4-(tolylsulphonyl)oxime], 2-aminomethyl-1,4-benzoquinone 4-[O-4-(tolylsulphonyl)oxime], 2-methoxymethyl-1,4-benzoquinone 4-[O-4-(tolylsulphonyl)oxime], 2-[(2-hydroxyethyl)amino]methyl-1,4-benzoquinone 4-[O-4-(tolylsulphonyl)oxime], 2-hydroxycarbonyl-1,4-benzoquinone 4-[O-4-(tolylsulphonyl)oxime],3-methyl-1,4-benzoquinone 4-[O-4-(tolylsulphonyl)oxime], 3-chloro-1,4-benzoquinone 4-[O-4-(tolylsulphonyl)oxime], 3-bromo-1,4-benzoquinone 4-[O-4-(tolylsulphonyl)oxime], 3-fluoro-1,4-benzoquinone 4-[O-4-(tolylsulphonyl)oxime], 3-hydroxymethyl-1,4-benzoquinone 4-[O-4-(tolylsulphonyl)oxime], 2,6-dichloro-1,4-benzoquinone 4-[O-4-(tolylsulphonyl)oxime], 2,6-dibromo-1,4-benzoquinone 4-[O-4-(tolylsulphonyl)oxime], 1,4-benzoquinone 4-(O-acetyloxime), 2-methyl-1,4-benzoquinone 4-(O-acetyloxime), 2-chloro-1,4-benzoquinone 4-(O-acetyloxime), 2-bromo-1,4-benzoquinone 4-(O-acetyloxime), 2-fluoro-1,4-benzoquinone 4-(O-acetyloxime), 2-hydroxymethyl-1,4-benzoquinone 4-(O-acetyloxime), 2-hydroxyethyl-1,4-benzoquinone 4-(O-acetyloxime), 2-aminomethyl-1,4-benzoquinone 4-(O-acetyloxime), 2-methoxymethyl-1,4-benzoquinone 4-(O-acetyloxime), 2-[(2-hydroxyethyl)amino]methyl-1,4-benzoquinone 4-(O-acetyloxime), 2-hydroxycarbonyl-1,4-benzoquinone 4-(O-acetyloxime), 3-methyl-1,4-benzoquinone 4-(O-acetyloxime), 3-chloro-1,4-benzoquinone 4-(O-acetyloxime), 3-bromo-1,4-benzoquinone 4-(O-acetyloxime), 3-fluoro-1,4-benzoquinone 4-(O-acetyloxime), 3-hydroxymethyl-1,4-benzoquinone 4-(O-acetyloxime), 2,6-dichloro-1,4-benzoquinone 4-(O-acetyloxime) and 2,6-dibromo-1,4-benzoquinone 4-(O-acetyloxime).

4. Agent according to Claim 1, **characterized in that** the quinonimine derivative of the formula (I) is chosen from 1,4-benzoquinone 4-[O-(methylsulphonyl)oxime], 2-methyl-1,4-benzoquinone 4-[O-(methylsulphonyl)oxime], 2-chloro-1,4-benzoquinone 4-[O-(methylsulphonyl)oxime], 2-bromo-1,4-benzoquinone 4-[O-(methylsulphonyl)oxime], 2-fluoro-1,4-benzoquinone 4-[O-(methylsulphonyl)oxime], 2-hydroxymethyl-1,4-benzoquinone 4-[O-(methylsulphonyl)oxime], 2-hydroxyethyl-1,4-benzoquinone 4-[O-(methylsulphonyl)oxime], 2-aminomethyl-1,4-benzoquinone 4-[O-(methylsulphonyl)oxime], 2-methoxymethyl-1,4-benzoquinone 4-[O-(methylsulphonyl)oxime], 2-[(2-hydroxyethyl)amino]methyl-1,4-benzoquinone 4-[O-(methylsulphonyl)oxime], 2-hydroxycarbonyl-1,4-benzoquinone 4-[O-(methylsulphonyl)oxime], 3-methyl-1,4-benzoquinone 4-[O-(methylsulphonyl)oxime], 3-chloro-1,4-benzoquinone 4-[O-(methylsulphonyl)oxime], 3-bromo-1,4-benzoquinone 4-[O-(methylsulphonyl)oxime], 3-fluoro-1,4-benzoquinone 4-[O-(methylsulphonyl)oxime], 3-hydroxymethyl-1,4-benzoquinone 4-[O-(methylsulphonyl)oxime], 2,6-dichloro-1,4-benzoquinone 4-[O-(methylsulphonyl)oxime], 2,6-dibromo-1,4-benzoquinone 4-[O-(methylsulphonyl)oxime], 2,5-dimethyl-1,4-benzoquinone 4-[O-(methylsulphonyl)oxime],2-isopropyl-5-methyl-1,4-benzoquinone 4-[O-(methylsulphonyl)oxime], 2-methyl-5-isopropyl-1,4-benzoquinone 4-[O-(methylsulphonyl)oxime] and 1,4-naphthoquinone 4-[O-(methylsulphonyl)oxime].

5. Agent according to one of Claims 1 to 4, **characterized in that** the amine or phenol is chosen from N-(3-dimethylaminophenyl)urea, 2,6-diamino-pyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, phenol, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylamino-phenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]-acetamide, 5-[(2-hydroxyethyl)amino]-4-methoxy-2-methylphenol, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

6. Agent according to one of Claims 1 to 5, **characterized in that** it comprises the quinonimine derivatives of the formula (I) and the amines, and/or phenols in the respective colour carrier mass (component (A1) or component (A2)) in each case in a total amount of from 0.02 to 20% by weight.

7. Agent according to one of Claims 1 to 6, **characterized in that** it comprises the quinonimine derivatives of the formula (I) and the amines and/or phenols in the ready-to-use colorant (A) in each case in a total amount of from 0.01 to 10% by weight.

8. Agent according to one of Claims 1 to 7, **characterized in that** it additionally comprises 0.02 to 20% by weight of a direct dye from the group of cationic and anionic dyes, disperse dyes, nitro dyes, azo dyes, quinone dyes and triphenylmethane dyes.

9. Agent according to one of Claims 1 to 8, **characterized in that** the ready-to-use colorant (A) has a pH of from 3 to 12.

10. Agent according to one of Claims 1 to 9, **characterized in that** it is a hair colorant.

11. Ready-to-use agent for colouring fibres (A), **characterized in that** it comprises at least one quinonimine derivative of the formula (I) and at least one compound from the group consisting of aromatic amines and phenols and has a pH of from 3 to 12; 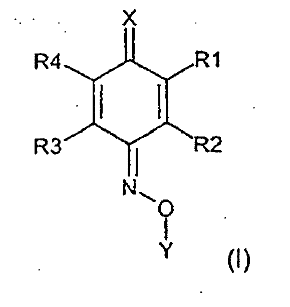 where, in formula (I),
**X** is oxygen or an NR group, where R is an optionally substituted (C₁-C₆)-alkyl group or an optionally substituted aromatic isocyclic or heterocyclic ring;
**Y** is a (C₁-C₆)-alkylsulphonyl group, an arylsulphonyl or an acetyl group;
**R1, R2, R3** and **R4** may be identical or different and, independently of one another, are hydrogen, a halogen atom, a (C₁-C₆)-alkyl group, a trifluoromethyl group, a (C₁-C₆)-alkyl group substituted by a halogen atom, a (C₁-C₆)-hydroxyalkyl group, a (C₂-C₆) -polyhydroxyalkyl group, a (C₁-C₆) -aminoalkyl group, a (C₁-C₆) -alkoxy group, a (C₁-C₆) -hydroxyalkoxy group, a (C₁-C₆) -aminoalkoxy group, a (C₁-C₆)-alkoxy-(C₁-C₆)-alkyl group, a (C₁-C₆)-alkoxy-(C₁-C₆)-alkoxy group, a (C₁-C₆)-hydroxyalkyl-(C₁-C₆)-aminoalkyl group, a nitro group, a cyano group, a carboxylic acid group, a carboxylic acid ester group or an optionally substituted aromatic ring system which is isocyclic or heterocyclic, where two adjacent radicals **R1** to **R4** together can also form a fused-on aromatic (isocyclic or heterocyclic) ring system.

12. Method of colouring keratin fibres in which the ready-to-use colorant (A) is prepared directly prior to use by mixing two components (A1) and (A2) and then applied to the hair and, after a contact time of from 5 to 60 minutes at a temperature of from 20 to 50°C, the hair is rinsed with water, optionally washed with a shampoo and then dried, **characterized in that** a colorant (A) according to one of Claims 1 to 10 is used.

13. Multicomponent kit for colouring hair, consisting of an agent of component (A1) according to one of Claims 1 to 9 and an agent of component (A2) according to one of Claims 1 to 9, and optionally an agent for adjusting the pH.

## Revendications

1. Agent pour la teinture de fibres (A), qui est préparé par mélange de deux composants (A1) et (A2), **caractérisé en ce que** le composant (A1) contient au moins un dérivé de quinone-imine de formule (I), et le composant (A2) contient au moins un composé du groupe formé par les amines aromatiques et les phénols ; 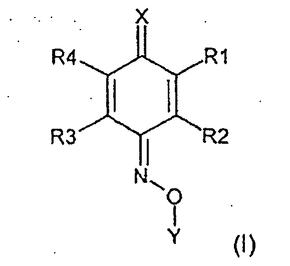 où, dans la formule (I)
X représente oxygène ou un groupe NR, R représentant un groupe (C₁-C₆)-alkyle le cas échéant substitué ou un cycle - isocyclique ou hétérocyclique - aromatique le cas échéant substitué ;
Y représente un groupe (C₁-C₆) -alkylsulfonyle, un groupe arylsulfonyle ou un groupe acétyle ;
R1, R2, R3 et R4 peuvent être identiques ou différents et représentent, indépendamment l'un de l'autre, hydrogène, un atome d'halogène, un groupe (C₁-C₆)-alkyle, un groupe trifluorométhyle, un groupe (C₁-C₆)-alkyle substitué par un atome d'halogène, un groupe (C₁-C₆)-hydroxyalkyle, un groupe (C₂-C₆)-polyhydroxyalkyle, un groupe (C₁-C₆)-aminoalkyle, un groupe (C₁-C₆)-alcoxy, un groupe (C₁-C₆) -hydroxyalcoxy, un groupe (C₁-C₆) - aminoalcoxy, un groupe (C₁-C₆) -alcoxy- (C₁-C₆) -alkyle, un groupe (C₁-C₆) -alcoxy- (C₁-C₆) -alcoxy, un groupe (C₁-C₆)-hydroxyalkyl-(C₁-C₆)-aminoalkyle, un groupe nitro, un groupe cyano, un groupe acide carboxylique, un groupe ester d'acide carboxylique ou un système cyclique (isocyclique ou hétérocyclique) aromatique, le cas échéant substitué, deux radicaux adjacents R1 à R4 pouvant également former, ensemble, un système cyclique aromatique condensé, qui est isocyclique ou hétérocyclique.

2. Agent selon la revendication 1, **caractérisé en ce que** dans la formule (I) X représente oxygène, Y représente un groupe méthylsulfonyle, un groupe phénylsulfonyle, un groupe 4-toluylsulfonyle, un groupe 4-chloro-phénylsulfonyle, un groupe acétyle ou un groupe chloroacétyle, et R1, R2, R3 et R4 peuvent être identiques ou différents et représentent, indépendamment l'un de l'autre, hydrogène, un atome d'halogène, un groupe (C₁-C₆)-alkyle, un groupe (C₁-C₆)-hydroxyalkyle, un groupe (C₁-C₆)-aminoalkyle, un groupe (C₁-C₆)-alcoxy, un groupe (C₁-C₆) -hydroxyalkyl- (C₁-C₆)-aminoalkyle, un groupe (C₁-C₆)-alcoxy-(C₁-C₆)-alkyle, un groupe acide carboxylique ou un système cyclique (isocyclique ou hétérocyclique) aromatique le cas échéant substitué, deux radicaux adjacents R1 à R4 pouvant également former ensemble un cycle (isocyclique ou hétérocyclique) aromatique condensé.

3. Agent selon la revendication 1, **caractérisé en ce que** le dérivé de quinone-imine de formule (I) est choisi parmi la 1,4-benzoquinone-4-[O-(méthylsulfonyl)-oxime], la 2-méthyl-1,4-benzoquinone-4-[O-(méthylsulfonyl)oxime], la 2-chloro-1,4-benzoquinone-4-[O-(méthylsulfonyl)oxime], la 2-bromo-1,4-benzoquinone-4-[O-(méthylsulfonyl)oxime], la 2-fluoro-1,4-benzoquinone-4-[O-(méthylsulfonyle)oxime], la 2-hydroxyméthyl-1,4-benzoquinone-4-[O-(méthylsulfonyl)-oxime], la 2-hydroxyéthyl-1,4-benzoquinone-4-(O-(méthylsulfonyl)oxime], la 2-aminométhyl-1,4-benzoquinone-4-[O-(méthylsulfonyl)oxime], la 2-méthoxyméthyl-1,4-benzoquinone-4-[O-(méthylsulfonyl)-oxime], la 2-[(2-hydroxyéthyl)-amino]méthyl-1,4-benzoquinone-4-(O-(méthylsulfonyl)oxime], la 2-hydroxycarbonyl-1,4-benzoquinone-4-[O-(méthylsulfonyl)-oxime], la 3-méthyl-1,4-benzoquinone-4-[O-(méthylsulfonyl)oxime], la 3-chloro-1,4-benzoquinone-4-[O-(méthylsulfonyl)oxime], la 3-bromo-1,4-benzoquinone-4-[O-(méthylsulfonyl)oxime], la 3-fluoro-1,4-benzoquinone-4-[O-(méthylsulfonyl)oxime], la 3-hydroxyméthyl-1,4-benzoquinone-4-[O-(méthylsulfonyl)-oxime], la 2,6-dichloro-1,4-benzoquinone-4-[O-(méthylsulfonyl)oxime], la 2,6-dibromo-1,4-benzoquinone-4-[O-(méthylsulfonyl)oxime], la 2,5-diméthyl-1,4-benzoquinone-4-[O-(méthylsulfonyl)oxime], la 2-isopropyl-5-méthyl-1,4-benzoquinone-4-[O-(méthylsulfonyl)oxime], la 2-méthyl-5-isopropyl-1,4-benzoquinone-4-[O-(méthylsulfonyl)oxime], la 1,4-naphtoquinone-4-[O-méthylsulfonyl)oxime], la 1-phénylimine-benzoquinone-4-[O-(méthylsulfonyl)oxime], la 1-méthylimine-benzoquinone-4-[O-(méthylsulfonyl)-oxime], la 1,4-benzoquinone-4-[O-(phénylsulfonyl)-oxime], la 2-méthyl-1,4-benzoquinone-4-[O-(phénylsulfonyl)oxime], la 2-chloro-1,4-benzoquinone-4-[O-(phénylsulfonyl)oxime], la 2-bromo-1,4-benzoquinone-4-[O-(phénylsulfonyl)oxime], la 2-fluoro-1,4-benzoquinone-4-[O-(phénylsulfonyl)oxime], la 2-hydroxyméthyl-1,4-benzoquinone-4-[O-(phénylsulfonyl)-oxime], la 2-hydroxyéthyl-1,4-benzoquinone-4-[O-(phénylsulfonyl)oxime], la 2-aminométhyl-1,4-benzoquinone-4-(O-(phénylsulfonyl)oxime], la 2-méthoxyméthyl-1,4-benzoquinone-4-[O-(phénylsulfonyl)-oxime], la 2-[(2-hydroxyéthyl)-amino]méthyl-1,4-benzoquinone-4-[O-(phénylsulfonyl)oxime], la 2-hydroxycarbonyl-1,4-benzoquinone-4-[O-(phénylsulfonyl)-oxime], la 3-méthyl-1,4-benzoquinone-4-[O-(phénylsulfonyl)oxime], la 3-chloro-1,4-benzoquinone-4-[O-(phénylsulfonyl)oxime], la 3-bromo-1,4-benzoquinone-4-[O-(phénylsulfonyl)oxime], la 3-fluoro-1,4-benzoquinone-4-[O-(phénylsulfonyl)oxime], la 3-hydroxyméthyl-1,4-benzoquinone-4-[O-(phénylsulfonyl)-oxime], la 2,6-dichloro-1,4-benzoquinone-4-[O-(phénylsulfonyl)oxime], la 2,6-dibromo-1,4-benzoquinone-4-[O-(phénylsulfonyl)oxime], la 1,4-benzoquinone-4-[O-4-(toluylsulfonyl)oxime], la 2-méthyl-1,4-benzoquinone-4-[O-4-(toluylsulfonyl)oxime], la 2-chloro-1,4-benzoquinone-4-[O-4-(toluylsulfonyl)-oxime], la 2-bromo-1,4-benzoquinone-4-[O-4-(toluylsulfonyl)oxime], la 2-fluoro-1,4-benzoquinone-4-[O-4-(toluylsulfonyl)oxime], la 2-hydroxyméthyl-1,4-benzoquinone-4-[O-4-(toluylsulfonyl)oxime], la 2-hydroxyéthyl-1,4-benzoquinone-4-[O-4-(toluylsulfonyl)-oxime], la 2-aminométhyl-1,4-benzoquinone-4-[O-4-(toluylsulfonyl)oxime], la 2-méthoxyméthyl-1,4-benzoquinone-4-[O-4-(toluylsulfonyl)oxime], la 2-[(2-hydroxyéthyl)-amino]méthyl-1,4-benzoquinone-4-[O-4-(toluylsulfonyl)oxime], la 2-hydroxycarbonyl-1,4-benzoquinone-4-[O-4-(toluylsulfonyl)oxime], la 3-méthyl-1,4-benzoquinone-4-[O-4-(toluylsulfonyl)oxime], la 3-chloro-1,4-benzoquinone-4-[O-4-(toluylsulfonyl)-oxime], la 3-bromo-1,4-benzoquinone-4-[O-4-(toluylsulfonyl)oxime], la 3-fluoro-1,4-benzoquinone-4-[O-4-(toluylsulfonyl)oxime], la 3-hydroxyméthyl-1,4-benzoquinone-4-[O-4-(toluylsulfonyl)oxime], la 2,6-dichloro-1,4-benzoquinone-4-[O-4-(toluylsulfonyl)-oxime], la 2,6-dibromo-1,4-benzoquinone-4-[O-4-(toluylsulfonyl)oxime], la 1,4-benzoquinone-4-(O-acétyloxime), la 2-méthyl-1,4-benzoquinone-4-(O-acétyloxime), la 2-chloro-1,4-benzoquinone-4-(O-acétyloxime), la 2-bromo-1,4-benzoquinone-4-(O-acétyloxime), la 2-fluoro-1,4-benzoquinone-4-(O-acétyloxime), la 2-hydroxyméthyl-1,4-benzoquinone-4-(O-acétyloxime), la 2-hydroxyéthyl-1,4-benzoquinone-4-(O-acétyloxime), la 2-aminométhyl-1,4-benzoquinone-4-(O-acétyloxime), la 2-méthoxyméthyl-1,4-benzoquinone-4-(O-acétyloxime), la 2-[(2-hydroxyéthyl)-amino]méthyl-1,4-benzoquinone-4-(O-acétyloxime), la 2-hydroxycarbonyl-1,4-benzoquinone-4-(O-acétyloxime), la 3-méthyl-1,4-benzoquinone-4-(O-acétyloxime), la 3-chloro-1,4-benzoquinone-4-(O-acétyloxime), la 3-bromo-1,4-benzoquinone-4-(O-acétyloxime), la 3-fluoro-1,4-benzoquinone-4-(O-acétyloxime), la 3-hydroxyméthyl-1,4-benzoquinone-4-(O-acétyloxime), la 2,6-dichloro-1,4-benzoquinone-4-(O-acétyloxime) et la 2,6-dibromo-1,4-benzoquinone-4-(O-acétyloxime).

4. Agent selon la revendication 1, **caractérisé en ce que** le dérivé de quinone-imine de formule (I) est choisi parmi la 1,4-benzoquinone-4-[O-(méthylsulfonyl)-oxime], la 2-méthyl-1,4-benzoquinone-4-[0-(méthylsulfonyl)oxime], la 2-chloro-1,4-benzoquinone-4-[O-(méthylsulfonyl)oxime], la 2-bromo-1,4-benzoquinone-4-[O-(méthylsulfonyl)oxime], la 2-fluoro-1,4-benzoquinone-4-[O-(méthylsulfonyl)oxime], la 2-hydroxyméthyl-1,4-benzoquinone-4-[O-(méthylsulfonyl)-oxime], la 2-hydroxyéthyl-1,4-benzoquinone-4-[O-(méthylsulfonyl)oxime], la 2-aminométhyl-1,4-benzoquinone-4-[O-(méthylsulfonyl)oxime], la 2-méthoxyméthyl-1,4-benzoquinone-4-[O-(méthylsulfonyl)-oxime], la 2-[(2-hydroxyéthyl)-amino]méthyl-1,4-benzoquinone-4-[O-(méthylsulfonyl)oxime], la 2-hydroxycarbonyl-1,4-benzoquinone-4-[O-(méthylsulfonyl)-oxime], la 3-méthyl-1,4-benzoquinone-4-[O-(méthylsulfonyl)oxime], la 3-chloro-1,4-benzoquinone-4-[O-(méthylsulfonyl)oxime], la 3-bromo-1,4-benzoquinone-4-[O-(méthylsulfonyl)oxime], la 3-fluoro-1,4-benzoquinone-4-[O-(méthylsulfonyl)oxime], la 3-hydroxyméthyl-1,4-benzoquinone-4-[O-(méthylsulfonyl)-oxime], la 2,6-dichloro-1,4-benzoquinone-4-[O-(méthylsulfonyl)oxime], la 2,6-dibromo-1,4-benzoquinone-4-[O-(méthylsulfonyl)oxime], la 2,5-diméthyl-1,4-benzoquinone-4-[O-(méthylsulfonyl)oxime], la 2-isopropyl-5-méthyl-1,4-benzoquinone-4-[O-(méthylsulfonyl)oxime], la 2-méthyl-5-isopropyl-1,4-benzoquinone-4-[O-(méthylsulfonyl)oxime] et la 1,4-naphtoquinone-4-[O-méthylsulfonyl)oxime].

5. Agent selon l'une quelconque des revendications 1 à 4, **caractérisé en ce que** l'amine ou le phénol est choisi parmi la N-(3-diméthylamino-phényl)-urée, la 2,6-diamino-pyridine, le 2-amino-4-[(2-hydroxyéthyl)-amino]-anisole, 2,4-diamino-1-fluoro-5-méthyl-benzène, le 2,4-diamino-1-méthoxy-5-méthyl-benzène, le 2,4-diamino-1-éthoxy-5-méthyl-benzène, le 2,4-diamino-1-(2-hydroxyéthoxy)-5-méthyl-benzène, le 2,4-di[(2-hydroxyéthyl)amino]-1,5-diméthoxy-benzène, la 2,3-diamino-6-méthoxy-pyridine, la 3-amino-6-méthoxy-2-(méthylamino)-pyridine, la 2,6-diamino-3,5-diméthoxy-pyridine, la 3,5-diamino-2,6-diméthoxy-pyridine, le 1,3-diamino-benzène, le 2,4-diamino-1-(2-hydroxyéthoxy)-benzène, le 1,3-diamino-4-(2,3-dihydroxypropoxy)-benzène, le 1,3-diamino-4-(3-hydroxypropoxy)-benzène, le 1,3-diamino-4-(2-méthoxyéthoxy)-benzène, le 2,4-diamino-1,5-di(2-hydroxyéthoxy)-benzène, le 1-(2-aminoéthoxy)-2,4-diamino-benzène, le 2-amino-1-(2-hydroxyéthoxy)-4-méthylamino-benzène, l'acide 2,4-diamino-phénoxy-acétique, la 3-[di(2-hydroxyéthyl)amino]aniline, le 4-amino-2-di[(2-hydroxyéthyl)amino]-1-éthoxy-benzène, le phénol, le 5-méthyl-2-(1-méthyléthyl)-phénol, la 3-[(2-hydroxyéthyl)amino]-aniline, la 3-[(2-aminoéthyl)amino]aniline, le 1,3-di(2,4-diaminophénoxy)-propane, le di(2,4-diaminophénoxy)-méthane, le 1,3-diamino-2,4-diméthoxy-benzène, le 2,6-bis(2-hydroxyéthyl)amino-toluène, le 4-hydroxyindole, le 3-diméthylamino-phénol, le 3-diéthylamino-phénol, le 5-amino-2-méthyl-phénol, le 5-amino-4-fluoro-2-méthyl-phénol, le 5-amino-4-méthoxy-2-méthyl-phénol, le 5-amino-4-éthoxy-2-méthyl-phénol, le 3-amino-2,4-dichloro-phénol, le 5-amino-2,4-dichloro-phénol, le 3-amino-2-méthyl-phénol, le 3-amino-2-chloro-6-méthyl-phénol, le 3-amino-phénol, le 2-[(3-hydroxyphényl)-amino]-acétamide, le 5-[(2-hydroxyéthyl)amino]-4-méthoxy-2-méthyl-phénol, le 5-[(2-hydroxyéthyl)amino]-2-méthyl-phénol, le 3-[(2-hydroxyéthyl)amino]-phénol, le 3-[(2-méthoxyéthyl)amino]-phénol, le 5-amino-2-éthyl-phénol, le 5-amino-2-méthoxy-phénol, le 2-(4-amino-2-hydroxyphénoxy)-éthanol, le 5-[(3-hydroxypropyl)amino]-2-méthyl-phénol, le 3-[(2,3-dihydroxypropyl)-amino]-2-méthyl-phénol, le 3-[(2-hydroxyéthyl)amino]-2-méthyl-phénol, la 2-amino-3-hydroxy-pyridine, la 2,6-dihydroxy-3,4-diméthylpyridine, le 5-amino-4-chloro-2-méthyl-phénol, le 1-naphtol, le 2-méthyl-1-naphtol, le 1,5-dihydroxy-naphtalène, le 1,7-dihydroxy-naphtalène, le 2,3-dihydroxy-naphtalène, le 2,7-dihydroxy-naphtalène, l'acétate de 2-méthyl-1-naphtol, le 1,3-dihydroxy-benzène, le 1-chloro-2,4-dihydroxy-benzène, le 2-chloro-1,3-dihydroxy-benzène, le 1,2-dichloro-3,5-dihydroxy-4-méthyl-benzène, le 1,5-dichloro-2,4-dihydroxy-benzène, le 1,3-dihydroxy-2-méthyl-benzène, le 3,4-méthylènedioxy-phénol, la 3,4-méthylènedioxy-aniline, le 5-[(2-hydroxyéthyl)amino]-1,3-benzodioxole, le 6-bromo-1-hydroxy-3,4-méthylènedioxy-benzène, l'acide 3,4-diamino-benzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxy-indole, la 5,6-dihydroxy-indoline, le 5-hydroxy-indole, le 6-hydroxy-indole, le 7-hydroxy-indole et la 2,3-indolinedione.

6. Agent selon l'une quelconque des revendications 1 à 5, **caractérisé en ce qu'**il contient les dérivés de quinone-imine de formule (I) ainsi que les amines et/ou les phénols dans chaque masse support de couleur (composant (A1) ou composant (A2)) à chaque fois en une quantité totale de 0,02 à 20% en poids.

7. Agent selon l'une quelconque des revendications 1 à 6, **caractérisé en ce qu'**il contient les dérivés de quinone-imine de formule (I) ainsi que les amines et/ou les phénols dans l'agent de teinture (A) prêt à l'emploi à chaque fois en une quantité totale de 0,01 à 10% en poids.

8. Agent selon l'une quelconque des revendications 1 à 7, **caractérisé en ce qu'**il contient en outre 0,02 à 20% en poids d'un colorant montant directement sur la fibre du groupe des colorants cationiques et anioniques, des colorants de dispersion, des colorants nitro, des colorants azo, des colorants quinone et des colorants triphénylméthane.

9. Agent selon l'une quelconque des revendications 1 à 8, **caractérisé en ce que** l'agent de teinture (A) prêt à l'emploi présente un pH de 3 à 12.

10. Agent selon l'une quelconque des revendications 1 à 9, **caractérisé en ce qu'**il s'agit d'un agent de teinture pour cheveux.

11. Agent prêt à l'emploi destiné à la teinture de fibres (A), **caractérisé en ce qu'**il contient au moins un dérivé de quinone-imine de formule (I) et au moins un composé du groupe constitué par les amines aromatiques et les phénols et présente un pH de 3 à 12 ; 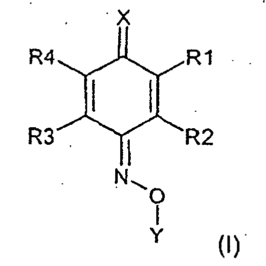 où, dans la formule (I)
X représente oxygène ou un groupe NR, R représentant un groupe (C₁-C₆)-alkyle le cas échéant substitué ou un cycle -isocyclique ou hétérocyclique - aromatique le cas échéant substitué ;
Y représente un groupe (C₁-C₆)-alkylsulfonyle, un groupe arylsulfonyle ou un groupe acétyle ;
R1, R2, R3 et R4 peuvent être identiques ou différents et représentent, indépendamment l'un de l'autre, hydrogène, un atome d'halogène, un groupe (C₁-C₆)-alkyle, un groupe trifluorométhyle, un groupe (C₁-C₆)-alkyle substitué par un atome d'halogène, un groupe (C₁-C₆)-hydroxyalkyle, un groupe (C₂-C₆)-polyhydroxyalkyle, un groupe (C₁-C₆)-aminoalkyle, un groupe (C₁-C₆)-alcoxy, un groupe (C₁-C₆) -hydroxyalcoxy, un groupe (C₁-C₆)-aminoalcoxy, un groupe (C₁-C₆)-alcoxy- (C₁-C₆) -alkyle, un groupe (C₁-C₆)-alcoxy-(C₁-C₆)-alcoxy, un groupe (C₁-C₆)-hydroxyalkyl-(C₁-C₆)-aminoalkyle, un groupe nitro, un groupe cyano, un groupe acide carboxylique, un groupe ester d'acide carboxylique ou un système cyclique aromatique le cas échéant substitué qui est isocyclique ou hétérocyclique, deux radicaux adjacents R1 à R4 pouvant également former, ensemble, un système cyclique aromatique condensé (isocyclique ou hétérocyclique).

12. Procédé pour la teinture de fibres kératiniques dans lequel l'agent de teinture prêt à l'emploi (A) est préparé juste avant l'utilisation par mélange de deux composants (A1) et (A2) puis est appliqué sur les cheveux et les cheveux sont rincés avec de l'eau après un temps d'action de 5 à 60 minutes à une température de 20 à 50°C, le cas échéant lavés avec un shampooing puis sont séchés, **caractérisé en ce qu'**on utilise un agent de teinture (A) selon l'une quelconque des revendications 1 à 10.

13. Kit à plusieurs composants pour la teinture des cheveux, constitué par un agent du composant (A1) selon l'une quelconque des revendications 1 à 9 et un agent du composant (A2) selon l'une quelconque des revendications 1 à 9, ainsi que le cas échéant un agent pour le réglage du pH.
